(19) Europäisches Patentamt
European Patent Office
Office européen des brevets

(11) Veröffentlichungsnummer : **0 343 437 B1**

(12) # EUROPÄISCHE PATENTSCHRIFT

(45) Veröffentlichungstag der Patentschrift : **18.03.92 Patentblatt 92/12**

(51) Int. Cl.$^5$ : **C07C 6/06**

(21) Anmeldenummer : **89108476.6**

(22) Anmeldetag : **11.05.89**

(54) **Verfahren zur Herstellung von Cycloalkadienen.**

(30) Priorität : **13.05.88 DE 3816453**

(43) Veröffentlichungstag der Anmeldung : **29.11.89 Patentblatt 89/48**

(45) Bekanntmachung des Hinweises auf die Patenterteilung : **18.03.92 Patentblatt 92/12**

(84) Benannte Vertragsstaaten : **BE CH DE ES FR GB IT LI NL SE**

(56) Entgegenhaltungen : **US-A- 4 668 836**

(73) Patentinhaber : **Consortium für elektrochemische Industrie GmbH**
**Zielstattstrasse 20**
**W-8000 München 70 (DE)**

(72) Erfinder : **Eberle, Hans-Jürgen, Dr.**
**Ambacher-Strasse 36**
**W-8000 München 71 (DE)**
Erfinder : **Csellich, Christine**
**Ottobrunner-Strasse 11**
**W-8025 Unterhaching (DE)**
Erfinder : **Zeitler, Norbert**
**Ganghoferstrasse 21**
**W-8000 München 2 (DE)**
Erfinder : **Meixner, Georg**
**Leitzachwerkstrasse 3**
**W-8152 Vagen (DE)**

**EP 0 343 437 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Cycloalkadienen in Flüssigphase durch eine Metathesereaktion in Gegenwart eines Trägerkatalysators auf Basis von $Re_2O_7/Al_2O_3$.

Es ist aus der US-A-4668836 bekannt, Cycloalkadiene durch Metathese von Cycloalkamonoenen in Gegenwart eines Trägerkatalysators auf Basis von $Re_2O_7/\gamma\text{-}Al_2O_3/SnR_4$ herzustellen.

Bei allen metathetischen Dimerisierungen von Cycloalkenen zu Cycloalkadienen fallen trotz optimaler Bedingungen höhere Cycloalkapolyene mit einem Polymerisierungsgrad größer oder gleich drei an. Bei der Dimerisierung von Cycloocten zu Cyclohexadecadien sind dies beispielsweise zwischen 50 und 70 Gew.-% (bezogen auf eingesetzte Menge Cycloocten) höherer Cyclopolyoctenylene. Eine sinnvolle Anwendung dieser Cyclopolyoctenylengemische ist bis jetzt nicht bekannt.

Gegenstand der Erfindung ist ein Verfahren zur Herstellung von Cycloalkadienen in Flüssigphase durch Metathesereaktion in Gegenwart eines Tragerkatalysators auf Basis von $Re_2O_7/Al_2O_3$, das dadurch gekennzeichnet ist, daß ein Cyclopolyoctenylen mit einem Polymerisierungsgrad größer oder gleich drei als 0,005 bis 0,2 molare Lösungen bei Verweilzeiten von 10 bis 600 Sekunden mit dem Trägerkatalysator in Kontakt gebracht wird.

Durch das erfindungsgemäße Verfahren lassen sich bei der Metathesereaktion von Cycloocten zu Cyclohexadecadien anfallende Cyclopolyoctenylene ebenfalls in einer Metathesereaktion zu Cycloalkadienen umsetzen. Überraschenderweise liegen hierbei die erreichbaren Ausbeuten bezüglich der Cycloalkadiene deutlich höher, als man es aus der Lage des thermodynamischen Gleichgewichts erwarten konnte. So können beim Abbau von Cyclopolyoctenylenen Ausbeuten bis zu 40% erreicht werden.

Die Ausbeuten liegen damit annähernd gleich, wie sie bei der Cyclooctenmetathese erhalten werden können, obgleich es sich bei der Cyclopolyoctenylenmetathese um eine Abbau-, bei der Cyclooctenmetathese um eine Aufbaureaktion handelt.

Durch das erfindungsgemäße Verfahren können die bei der Cyclooctenmetathese anfallenden Cyclopolyoctenylene zu wesentlich wertvolleren Cycloalkadienen verwertet werden.

Mehr noch, da es sich gezeigt hat, daß bei Aufbau und Abbau fast gleichartiges Reaktionsverhalten vorliegt, können zur Herstellung von Cycloalkadienen auch Cycloalkamonoen/Cyclopolyoctenylen-Gemische verwendet werden.

Somit ist ein weiterer Gegenstand der Erfindung ein Verfahren zur Herstellung von Cycloalkadienen in Flüssigphase durch Metathesereaktion in Gegenwart eines Trägerkatalysators auf Basis von $Re_2O_7/Al_2O_3$, das dadurch gekennzeichnet ist, daß ein Cycloalkamonoen/Cyclopolyoctenylen-Gemisch, wobei das Cyclopolyoctenylen einen Polymerisierungsgrad größer oder gleich drei aufweist, als 0,005 bis 0,2 molare Lösungen bei Verweilzeiten von 10 bis 600 Sekunden mit dem Trägerkatalysator in Kontakt gebracht wird.

Die Angabe Mol bei den Konzentrationen der Lösungen bezieht sich im Sinne der Erfindung auf errechnete Cycloocteneinheiten, die sich ergeben, wenn man die Cyclopolyoctenylene in die Monomeren unterteilt, sowie bei der Verwendung von Cycloalkamonoen/Cyclopolyoctenylen-Gemischen zu den zugegebenen Cycloalkamonoeneinheiten addiert.

Bei den erfindungsgemäßen Verfahren werden 0,01 bis 0,05 molare Lösungen bevorzugt. Bevorzugte Verweilzeiten sind 10 bis 200 Sekunden.

Verwendete Cyclopolyoctenylene entsprechen der allgemeinen Formel

$$\left[-CH=CH-(CH_2)_6-\right]_n$$

und weisen einen Polymerisierungsgrad n von zumindest 3, vorzugsweise 3 bis 50, insbesondere 3 bis 20, auf.

Werden Cyclopolyoctenylen/Cycloalkamonoen-Gemische verwendet, so ist ein Anteil an Cycloalkamonoenen von 1 bis 90 Gew.-% (bezogen auf Gesamtgewicht des Gemisches), insbesondere von 1 bis 50 Gew.-%, bevorzugt.

Bevorzugt verwendete Cycloalkamonoene sind Cyclopenten, Cyclohepten, Cycloocten, Cyclodecen und Cyclododecen. Insbesondere werden Cycloocten oder Cyclohepten eingesetzt.

Es werden insbesondere solche Trägerkatalysatoren eingesetzt, die $\gamma\text{-}Al_2O_3$ als Trägermaterial aufweisen. Die spezifische Oberfläche des Trägermaterials beträgt vorzugsweise 100 bis 300 $m^2/g$ nach BET.

Das Trägermaterial wird insbesondere in Form von Hohlsträngen, Kugeln, Zylindern, Würfeln, Kegeln und dergleichen eingesetzt.

Der Gewichtsanteil an $Re_2O_7$ am Gesamtgewicht des Katalysators beträgt vorzugsweise 3 bis 20 Gew.-%, insbesondere 3 bis 7 Gew.-%.

Es ist im Rahmen der Erfindung bevorzugt, solche Trägerkatalysatoren auf Basis von $Re_2O_7/\gamma\text{-}Al_2O_3$ einzusetzen, die noch mit Co-Katalysatoren beladen sind. Die Menge an Co-Katalysator wird so bemessen, daß das molare Verhältnis von Re zu Co-Katalysator 50:1 bis 1:2, vorzugsweise 5:1 bis 1:2, beträgt.

Bevorzugte Co-Katalysatoren sind Verbindungen der Formel $MRR'_m$,

wobei

R für ein Alkyl- oder Arylrest und

R′ für Halogen-, Alkyl- oder Arylrest steht, und

M für m gleich 2 Aluminium und für m gleich 3 Zinn oder Blei bedeutet.

Beispiele für Reste R und R′ sind der Methyl-, Ethyl-, n-Butyl-und Phenylrest. Ein Beispiel für einen Halogenrest ist der Chlorrest.

Insbesonders verwendete Co-Katalysatoren sind Blei- oder Zinnverbindungen der oben angegebenen Formel. Beispiele hierfür sind Zinntetramethyl, Zinntetraethyl, Zinntetra-n-butyl, Bleitetramethyl, Bleitetraethyl und Bleitetra-n-butyl.

Die Herstellung der einzusetzenden Trägerkatalysatoren ist an sich bekannt, sie erfolgt beispielsweise durch Imprägnierung des Trägermaterials mit einer wäßrigen Lösung von Ammoniumperrhenat und anschließender thermischer Behandlung des Guts, wobei die Rheniumverbindung in das Oxid überführt wird.

Die Behandlung des $Re_2O_7$/ $Al_2O_3$-Kontakts mit Co-Katalysator erfolgt zweckmäßigerweise durch Behandlung des Katalysatormaterials mit Lösungen der entsprechenden Verbindung in aliphatischen oder aromatischen Kohlenwasserstoffen. Beispiele für derartige Lösungsmittel sind Metathese-inerte Lösungsmittel, die auch zur Verdünnung der Ausgangssubstanzen eingesetzt werden, wie Alkane beispielsweise Pentan, Hexan, Heptan, Cyclopentan, Cyclohexan, Petrolether vom Siedebereich von 30 bis 60 °C, chlorierte Kohlenwasserstoffe beispielsweise Methylenchlorid, Chloroform, Tetrachlorkohlenstoff oder Aromaten beipielsweise Chlorbenzol, m-Dichlorbenzol.

Vorzugsweise wird in einer Festbettanordnung gearbeitet, wobei gelöstes Cyclopolyoctenylen ggf. im Gemisch mit Cycloalkamonoen das Katalysatorbett als Flüssigphase durchströmt. Die Strömungsgeschwindigkeit wird erfindungsgemäß so eingestellt, daß Verweilzeiten von 10 bis 600 Sekunden, vorzugsweise 10 bis 200 Sekunden, eingehalten werden.

Die Reaktionstemperaturen liegen vorzugsweise im Bereich von 0 °C bis 100 °C. Vorzugsweise wird die Reaktion bei Drucken von 0,1 bis 100 bar, insbesondere 1 bis 10 bar, durchgeführt. Beim Arbeiten unter erhöhtem Druck kann die Reaktionstemperatur durchaus erhöht werden.

Beispielsweise wird zur Durchführung des Verfahrens ein mit Katalysator gefüllter Rohrreaktor in vertikaler Anordnung von einer Lösung von Cyclopolyoctenylenen und ggf. Cycloalkamonoenen durchströmt. Das den Reaktor verlassende Reaktionsgemisch wird einer Destillationsvorrichtung aufgegeben und in seine Komponenten getrennt. Das Zielprodukt fällt als relativ hochsiedende Fraktion an. In der Praxis wird zumeist ein kontinuierlicher Betrieb aufrechterhalten, wobei das als Leichtsieder anfallende Lösungsmittel sowie ggf. nicht umgesetztes Cycloalkamonoen im Kreislauf geführt und nach Beschickung mit neuem Ausgangsprodukt in den Reaktor zurückgeleitet wird.

Nach dem erfindungsgemäßen Verfahren gelingt es, makrocyclische Alkadiene mit vorzugsweise 15 bis 20 Kohlenstoffatomen, insbesondere Cyclohexadecadien-1.9 und Cyclopentadecadien-1.8, in guten Ausbeuten herzustellen. Die Ausbeuten der Reaktion liegen, bezogen auf die Gesamtmenge an eingesetztem Cycloolefin, bei bis zu 40 %. Es gelingt damit, chemisch an sich schwer zugängliche Verbindungen in wirtschaftlicher Weise herzustellen. Die Zielprodukte finden insbesondere Verwendung auf dem Riechstoffsektor, beispielsweise als Ausgangsprodukte für die Herstellung von Moschus-Riechstoffen.

Beispiel 1

Herstellung von Cyclohexadecadien-1.9 durch Metathese eines Cyclopolyoctenylen-Gemisches

Es wurde ein Rohrreaktor (Länge 70 cm, Durchmesser 8 cm) in vertikaler Anordnung verwendet, der mit 1,8 kg Trägerkatalysator beaufschlagt war. Der Katalysator enthielt 3,5 Gew.-% $Re_2O_7$ und 1,3 Gew.-% Zinntetramethyl. Als Trägermaterial wurde ein zu Zylindern geformtes $\gamma\text{-}Al_2O_3$ mit einer spezifischen Oberfläche nach BET von 190 $m^2/g$ verwendet. Durch das Katalysatorbett wurde eine 0,0052 molare Lösung eines Cyclopolyoctenylengemisches in Pentan geleitet. Die Zusammensetzung des eingesetzten Cyclopolyoctenylengemisches ist in Tab. 1 wiedergegeben. Die Temperatur im Katalysatorbett betrug 20 °C ; der Druck 1,03 bar. Die mittlere Verweilzeit des Reaktionsgemisches betrug 80 Sekunden.

Tab. 1 Zisammensetzung des Cyclopolyoctenylengemisches in Gew.-%

| Polymerisierungsgrad | Anteil (Gew.-%) |
|---|---|
| 3 | 42,5 |
| 4 | 23,8 |
| 5 | 13,3 |
| 6 | 7,9 |
| 7 und größer | 10,1 |

Das den Reaktor verlassende Reaktionsgemisch wurde einer Destillationsvorrichtung zugeführt, wobei n-Pentan abdestilliert und nach Beladen mit frischem Cyclopolyoctenylengemisch wieder in den Reaktor zurückgeführt wurde. Das nach dem Abdestillieren des n-Pentan verbleibende Reaktionsprodukt wurde gaschromatographisch analysiert.

Das Produktgemisch setzte sich demnach aus 39,5 Gew.-% Cyclohexadecadien und 60,5 Gew.-% höheren Cyclopolyoctenylenen zusammen.

Beispiel 2

Herstellung von Cyclohexadecadien-1.9 durch Metathese eines Cyclopolyoctenylen/Cycloocten-Gemisches

Es wurde ein Rohrreaktor (Länge 155 cm, Durchmesser 25 cm) in vertikaler Anordnung verwendet, der mit 38 kg Trägerkatalysator gefüllt war. Die Zusammensetzung des Katalysators war wie in Beispiel 1 beschrieben.

Durch das Katalysatorbett wurde eine 0,022 molare Lösung (bezogen auf Cycloocteneinheiten) eines Cyclopolyoctenylen/ Cycloocten-Gemisches in n-Pentan geleitet. Die Zusammensetzung Cyclopolyoctenylen zu Cycloocten betrug 3:1 (in Gewichtsanteilen). Das verwendete Cyclopolyoctenylen wies die in Tab. 1 in Beispiel 1 wiedergegebene Zusammensetzung auf. Die mittlere Verweilzeit des Reaktionsgemisches betrug 95 Sekunden, die Temperatur im Katalysatorbett 23 °C und der Druck 1,05 bar.

Das den Reaktor verlassende Reaktionsgemisch wurde einer Destillationsvorrichtung zugeführt, wobei das Lösungsmittel abdestilliert und nach Beladen mit neuem Cyclopolyoctenylen/Cycloocten-Gemisch der oben genannten Zusammensetzung wieder in den Reaktor zurückgeführt wurde.

Insgesamt wurde auf diese Weise 274,4 kg Cycloolefingemisch umgesetzt. Eine Aufdestillation des Reaktionsgemisches ergab 68,6 kg Cyclohexadecadien, entsprechend einer Ausbeute von 25 % bezogen auf die eingesetzte Gesamtcycloolefinmenge.

## Patentansprüche

1. Verfahren zur Herstellung von Cycloalkadienen in Flüssigphase durch Metathesereaktion in Gegenwart eines Trägerkatalysators auf Basis von $Re_2O_7/Al_2O_3$, dadurch gekennzeichnet, daß ein Cyclopolyoctenylen mit einem Polymerisierungsgrad größer oder gleich drei als 0,005 bis 0,2 molare Lösungen bei Verweilzeiten von 10 bis 600 Sekunden mit dem Trägerkatalysator in Kontakt gebracht wird.

2. Verfahren nach Anspruch 1, dadurch gekennzeichnet, daß ein Cycloalkamonoen/Cyclopolyoctenylen-Gemisch verwendet wird.

3. Verfahren nach Anspruch 1 oder 2, dadurch gekennzeichnet, daß 0,01 bis 0,05 molare Lösungen verwendet werden.

4. Verfahren nach einem oder mehreren der Ansprüche 1 bis 3, dadurch gekennzeichnet, daß die Verweilzeiten 10 bis 200 Sekunden betragen.

5. Verfahren nach einem oder mehreren der Ansprüche 1 bis 4, dadurch gekennzeichnet, daß Cyclopolyoctenylene mit einem Polymerisierungsgrad von 3 bis 50 eingesetzt werden.

6. Verfahren nach einem oder mehreren der Ansprüche 2 bis 5, dadurch gekennzeichnet, daß der Anteil an Cycloalkamonoenen im Cyclopolyoctenylen/Cyloalkamonoen-Gemisch 1 bis 90 Gew.-% (bezogen auf das Gesamtgewicht des Gemisches) beträgt.

7. Verfahren nach einem oder mehreren der Ansprüche 1 bis 6, dadurch gekennzeichnet, daß Trägerkatalysatoren eingesetzt werden, die $\gamma$-$Al_2O_3$ als Trägermaterial aufweisen, wobei die spezifische Oberfläche des

Trägermaterials 100 bis 300 $m^2/g$ nach BET beträgt .

8. Verfahren nach einem oder mehreren der Ansprüche 1 bis 7, dadurch gekennzeichnet, daß der Gewichtsanteil an $Re_2O_7$ am Gesamtgewicht des Katalysators 3 bis 20 Gew.-% beträgt.

9. Verfahren nach einem oder mehreren der Ansprüche 1 bis 8, dadurch gekennzeichnet, daß Trägerkatalysatoren eingesetzt werden, die mit Co-Katalysatoren beladen sind, wobei die Menge an Co-Katalysator so bemessen wird, daß das molare Verhältnis von Re zu Co-Katalysator 50:1 bis 1:2 beträgt.

10. Verfahren nach einem oder mehreren der Ansprüche 1 bis 9, dadurch gekennzeichnet, daß als Co-Katalysatoren Verbindungen der Formel

$$MRR'_m ,$$

wobei
R für Alkyl- oder Arylrest und
R′ für Halogen-, Alkyl- oder Arylrest steht, und
M für m gleich 2 Aluminium und für m gleich 3 Zinn oder Blei bedeutet, eingesetzt werden.

## Claims

1. Process for the preparation of cycloalkadienes in liquid phase by a metathesis reaction in the presence of a supported catalyst based on $Re_2O_7/Al_2O_3$, characterised in that a cyclopolyoctenylene having a polymerisation degree of greater than or equal to three in the form of 0.005 to 0.2 molar solutions at residence times of 10 to 600 seconds is brought into contact with the supported catalyst.

2. Process according to Claim 1, characterised in that a cycloalkamonoene/cyclopolyoctenylene mixture is used.

3. Process according to Claim 1 or 2, characterised in that 0.01 to 0.05 molar solutions are used.

4. Process according to one or more of Claims 1 to 3, characterised in that the residence times are 10 to 200 seconds.

5. Process according to one or more of Claims 1 to 4, characterised in that cyclopolyoctenylenes which have a polymerisation degree of 3 to 50 are used.

6. Process according to one or more of Claims 2 to 5, characterised in that the cycloalkamonoene content in the cyclopolyoctenylene/cycloalkamonoene mixture is 1 to 90% by weight, (relative to the total weight of the mixture) [sic].

7. Process according to one or more of Claims 1 to 6, characterised in that supported catalysts are used which have $\gamma$-$Al_2O_3$ as the support material, in which the specific surface area of the support material is 100 to 300 $m^2/g$ according to BET.

8. Process according to one or more of Claims 1 to 7, characterised in that the relative weight of $Re_2O_7$ in the total weight of the catalyst is 3 to 20% by weight.

9. Process according to one or more of Claims 1 to 8, characterised in that supported catalysts are used which are charged with co-catalysts, in which the amount of co-catalyst is such that the molar ratio of Re to co-catalyst is 50:1 to 1:2.

10. Process according to one or more of Claims 1 to 9, characterised in that the co-catalysts used are compound [sic] of the formula

$$MRR'_m$$

in which
R stands for an alkyl or aryl radical and
R′ for a halogen, alkyl or aryl radical and
M denotes aluminium, if m is 2, and tin or lead, if m is 3.

## Revendications

1. Procédé de préparation de cycloalcadiènes en phase liquide par une réaction de métathèse en présence d'un catalyseur sur support à base de $Re_2O_7/Al_2O_3$, procédé caractérisé en ce que l'on met en contact avec le catalyseur pendant une durée de 10 à 600 secondes un cyclopolyocténylène d'un degré de polymérisation égal ou supérieur à 3, en solution 0,005 à 0,2 molaire.

2. Procédé selon la revendication 1, caractérisé en ce que l'on traite un mélange de cycloalcamonoènes et de cyclopolyocténylènes.

3. Procédé selon la revendication 1 ou 2, caractérisé en ce que l'on opère sur des solutions 0,01 à 0,05 molaires.

4. Procédé selon une ou plusieurs des revendications 1 à 3, caractérisé en ce que les temps de séjour des mélanges de réactions sont de 10 à 200 secondes.

5. Procédé selon une ou plusieurs des revendications 1 à 4, caractérisé en ce que l'on traite des cyclopolyocténylènes ayant un degré de polymérisation de 3 à 50.

6. Procédé selon une ou plusieurs des revendications 2 à 5, caractérisé en ce que la teneur en cycloalcamonoènes du mélange de cyclopolyocténylènes et de cycloalcamonoènes est de 1 à 90% du poids total du mélange.

7. Procédé selon une ou plusieurs des revendications 1 à 6, caractérisé en ce que l'on utilise des catalyseurs sur un support de $Al_2O_3$ γ, dont la surface spécifique BET est de 100 à 300 $m^2/g$.

8. Procédé selon une ou plusieurs des revendications 1 à 7, caractérisé en ce que la proportion de $Re_2O_7$ est de 3 à 20% du poids total du catalyseur.

9. Procédé selon une ou plusieurs des revendications 1 à 8, caractérisé en ce que l'on utilise des catalyseurs chargés de cocatalyseurs dans une proportion donnant un rapport molaire de Re au cocatalyseur compris entre 50:1 et 1:2.

10. Procédé selon une ou plusieurs des revendications 1 à 9, caractérisé en ce que le cocatalyseur est un composé de formule

$$MRR'_m$$

dans laquelle
R représente un alkyle ou un aryle,
R' un halogène, un alkyle ou un aryle, et
M l'aluminium si m = 2 et l'étain ou le plomb si m = 3.